(19) Europäisches Patentamt
European Patent Office
Office européen des brevets

(11) Veröffentlichungsnummer: **0 058 410**
**B1**

(12) # EUROPÄISCHE PATENTSCHRIFT

(45) Veröffentlichungstag der Patentschrift: **13.06.84**

(51) Int. Cl.³: **A 23 L 1/236**, A 61 J 3/10

(21) Anmeldenummer: **82101040.2**

(22) Anmeldetag: **12.02.82**

(54) **Acesulfamhaltige Zusammensetzung, Tabletten auf Basis dieser Zusammensetzung und Verfahren zu ihrer Herstellung.**

(30) Priorität: **18.02.81 DE 3105813**

(43) Veröffentlichungstag der Anmeldung: **25.08.82 Patentblatt 82/34**

(45) Bekanntmachung des Hinweises auf die Patenterteilung: **13.06.84 Patentblatt 84/24**

(84) Benannte Vertragsstaaten: **AT BE CH DE FR GB IT LI NL SE**

(56) Entgegenhaltungen:
**CH-A- 551 193**
**DE-C- 2 001 017**

(73) Patentinhaber: **HOECHST AKTIENGESELLSCHAFT**
**Postfach 80 03 20**
**D-6230 Frankfurt am Main 80 (DE)**

(72) Erfinder: **Fülberth, Werner, Dr.**
**Theodor-Storm-Strasse 11**
**D-6233 Kelkheim (Taunus) (DE)**
Erfinder: **Sickmüller, Alfred, Dr.**
**Schweinfurter Weg 72**
**D-6000 Frankfurt am Main 70 (DE)**
Erfinder: **Stammberger, Willi, Dr.**
**Sachsenring 15**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 058 410 B1

Jouve, 18, rue St-Denis, 75001 Paris, France

**Beschreibung**

Die Erfindung betrifft eine physiologisch verträgliche Zusammensetzung enthaltend Acesulfam, für Süßstofftabletten übliche Tablettierhilfsstoffe und Gelatine als Trockenbindemittel. Die Zusammensetzung eignet sich zum Verpressen zu Tabletten.

In den vorstehenden und folgenden Ausführungen wird unter Acesulfam die freie Säure sowie die physiologisch verträglichen Salze verstanden.

Acesulfam ist ein Süßstoff der Formel

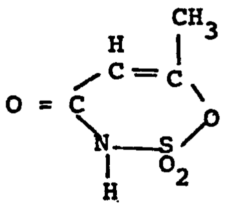

Er wird in der DE-C 2 001 017 beschrieben.

Die physikalischen Eigenschaften von Acesulfam, d. h. die Korngröße, das Schüttelvolumen sowie die Kristallform gestatten es nicht, Tabletten von ausreichender Qualität ohne Bindemittel herzustellen.

Von Acesulfam enthaltenden Tabletten wird, wie von allen Süßstofftabletten, gefordert, daß sie einerseits ausreichend mechanisch stabil sind (hohe Bruchfestigkeit und geringer Abrieb nach mechanischer Belastung), andererseits, trotz der guten mechanischen Festigkeit auch in kalten Getränken schnell und klar löslich sind.

Eine rasche Auflösung der Süßstofftabletten wird üblicherweise dadurch erreicht, daß die Zusammensetzung eine Säure und eine lösliche $CO_2$-liefernde Verbindung enthält, also eine Brausemischung, die sich in Gegenwart von Wasser unter Bildung von $CO_2$ auflöst.

Es ist bereits beschrieben worden, daß die mechanische Stabilität von Tabletten durch Bindemittel beeinflußt werden kann (Voigt, Lehrbuch der pharmazeutischen Technologie, VEB Verlag Volk und Gesundheit (1973), S. 180).

Gelatine erfüllt in hohem Maße die Funktion als Bindemittel und kann auch im Sinne der Verordnung über diätetische Lebensmittel (Januar 1978) bei Süßstofftabletten eingesetzt werden. Der Verwendung von Gelatine zur Herstellung von Süßstofftabletten steht jedoch entgegen, daß Gelatine nach dem Stand der Technik nur als Lösung bei der Feuchtgranulation eingesetzt wird (vgl. z. B. Hager, Handbuch der pharmazeutischen Praxis, 4. Neuausgabe, Band VII, S. 702 ; Voigt, s. oben, S. 160, 180, 181) und eine Feuchtgranulation mit Wasser bzw. unter Verwendung einer wäßrigen Gelatinelösung wegen des Gehaltes der Tablettenmischung an $CO_2$-liefernder Verbindung und Säure in der Regel nicht in Frage kommt. Es hat sich gezeigt, daß mit Einsatz der Feuchtgranulation bei der Herstellung von Acesulfam-Tabletten erhebliche Zerfallsbeeinträchtigung einhergeht. Eine Feuchtgranulation mit organischen Lösungsmitteln bzw. einer Lösung von Gelatine in organischen Lösungsmitteln ist prinzipiell durchführbar, aber wegen der damit verbundenen Umweltbelastung und wegen des aufwendigen Herstellungsprozesses wenig geeignet.

Es hat sich nun überraschenderweise gezeigt, daß Gelatine in Pulverform (Teilchengröße $< 300\ \mu m$) in einer Tablettenmischung aus Acesulfam, Natriumhydrogencarbonat und gegebenenfalls einer Säure selbst in der sehr niedrigen Konzentration von 0,7 %, die unter der allgemein üblichen Konzentration liegt, noch die Funktion als Bindemittel erfüllt, wenn sie in trockener Form zugemischt wird. Die mechanische Stabilität der Tabletten wird verbessert, und die Zerfallszeit der Tabletten wird nicht nachteilig beeinflußt.

Die Erfindung betrifft daher eine Zusammensetzung enthaltend neben Acesulfam oder einem physiologisch verträglichen Acesulfam-Salz, einer wasserlöslichen $CO_2$-liefernden Verbindung und gegebenenfalls einer physiologisch verträglichen festen Säure Gelatinepulver als Trockenbindemittel.

Die Zusammensetzungen enthalten vorzugsweise Acesulfam als freie Säure oder in Form des Kaliumsalzes. Im letzteren Fall ist es zweckmäßig, eine Säure zuzusetzen. Von den physiologisch verträglichen Säuren sind insbesondere Weinsäure und Zitronensäure geeignet. Als $CO_2$-liefernde Verbindungen kommen z. B. in Betracht : Natrium- und Kaliumhydrogencarbonat, Magnesiumcarbonat, Calciumhydrogencarbonat und Natriumglykolcarbonat sowie Gemische dieser Verbindungen. Bevorzugt ist die Verwendung von Natriumhydrogencarbonat.

Das Trockenbindemittel Gelatine wird in Pulverform eingesetzt, die Teilchengröße liegt zweckmäßigerweise unter 300 μm. Die Gelatine sollte Arzneibuchqualität besitzen (z. B. entspr. US P XX). Der Gehalt an Gelatine in der Zusammensetzung liegt vorzugsweise zwischen 0,4 bis 5 Gewichts-%, das Gewichtsverhältnis von Süßstoff zu Natriumhydrogencarbonat ist 1 : 5, vorzugsweise 1 : 1 bis 1 : 2.

Die erfindungsgemäße Zusammensetzung kann sowohl nach vorheriger Trockengranulation als auch, bei Einsatz geeigneter Tablettenmaschinen, direkt zu Tabletten verpreßt werden.

Die Erfindung betrifft daher auch Tabletten enthaltend die erfindungsgemäße Zusammensetzung sowie Verfahren zur Herstellung dieser Tabletten.

Eine Tablette enthält vorzugsweise 20 mg Süßstoff, das Gesamtgewicht liegt bevorzugt zwischen 40 und 70 mg.

Das Verfahren zur Herstellung der Tabletten ist dadurch gekennzeichnet, daß man die erfindungsgemäße Zusammensetzung entweder direkt oder nach vorheriger Trockengranulation zu Tabletten verpreßt.

Das Verfahren wird vorzugsweise wie folgt durchgeführt : Man mischt den Süßstoff mit dem oder den Hilfsstoffen ($CO_2$-liefernde Verbindung, Säure), unterwirft das Gemisch einer Trockengranulation, vermischt das Granulat mit dem Gelatinepulver und verpreßt es zu Tabletten. Man kann auch den Süßstoff mit dem oder den Hilfsstoffen und dem Gelatinepulver mischen und das Gemisch direkt zu Tabletten verpressen.

Die so hergestellten Acesulfam-« Brausetabletten » lösen sich trotz des Zusatzes eines Trockenbindemittels schnell (s. Tabelle) und klar in Wasser, Tee, Kaffee und anderen Getränken. Sie sind bezüglich mechanischer Stabilität den bindemittelfreien Tabletten überlegen (s. Tabelle I).

Beispiel 1

| | |
|---|---|
| 1) Acesulfam-Kalium | 20,0 mg |
| 2) Natriumhydrogencarbonat | 20,0 mg |
| 3) Weinsäure | 20,0 mg |
| 4) Gelatine | 1,0 mg |
| | 61,0 mg |

Herstellung

Die Substanzen 1)-4) werden gemischt und z. B. zu 5 mm biplanen Tabletten direkt verpreßt. Die resultierenden Tabletten wurden im Vergleich zu Tabletten, die ohne Gelatine hergestellt wurden, auf Zerfalls- und Laminationseigenschaften sowie auf Abrieb (Friabilität) untersucht. Die Ergebnisse sind in Tabelle I zusammengestellt.

Beispiel 2

| | |
|---|---|
| 1) Acesulfam-Kalium | 20,0 mg |
| 2) Natriumhydrogencarbonat | 20,0 mg |
| 3) Citronensäure | 5,0 mg |
| 4) Gelatine | 0,3 mg |
| | 45,3 mg |

Herstellung

Die Substanzen 1)-2) und 3) werden gemischt, kompaktiert und anschließend durch ein 0,8 mm Sieb passiert.

Das so erhaltene Granulat wird mit Substanz 4) gemischt und zu Tabletten von 5 mm Durchmesser verpreßt.

Beispiel 3

| | |
|---|---|
| 1) Acesulfam (freie Säure) | 20,00 mg |
| 2) Natriumhydrogencarbonat | 32,00 mg |
| 3) Gelatine | 1,00 mg |
| | 53,00 mg |

Die Substanzen 1)-3) werden gemischt und zu Tabletten von 5 mm Durchmesser verpreßt.
Die Prüfungsergebnisse sind in Tabelle I zusammengestellt.

Tabelle I

| Tabletten nach | Beispiel 1 | Beispiel 2 | Beispiel 3 | Beispiel 1, jedoch ohne Gelatine |
|---|---|---|---|---|
| Zerfallszeit in Wasser von 37 °C | 20-50" | 20-50" | 20-30" | 20-50" |

(Fortsetzung)

| Tabletten nach | Beispiel 1 | Beispiel 2 | Beispiel 3 | Beispiel 1, jedoch ohne Gelatine |
|---|---|---|---|---|
| Laminations-tendenz | nicht vorhanden | nicht vorhanden | nicht vorhanden | ausgeprägt |
| Abrieb * (Roche Friabilator) | 0-2 % | 0-2 % | 0-2 % | 2-20 % |
| Anzahl der zerbrochenen Tabletten beim Abriebstest * (mit dem Roche Friabilator) | 0 | 0 | 0 | 2-20 |

* 20 Tabletten werden 10 Minuten einer Rotation unterworfen von ca. 24 UpM.

**Ansprüche** (für die Vertragsstaaten : BE, CH, DE, FR, GB, IT, LI, NL, SE)

1. Zusammensetzung enthaltend Acesulfam oder ein physiologisch verträgliches Acesulfam-Salz, eine wasserlösliche, $CO_2$-liefernde Verbindung und gegebenenfalls eine physiologisch verträgliche, feste Säure, dadurch gekennzeichnet, dass sie Gelatinepulver als Trockenbindemittel enthält.
2. Zusammensetzung gemäß Anspruch 1, dadurch gekennzeichnet, daß sie als $CO_2$-liefernde Verbindung Natriumhydrogencarbonat enthält.
3. Zusammensetzung gemäß Anspruch 1 oder 2, dadurch gekennzeichnet, daß der Gehalt an Gelatinepulver 0,4 bis 5 % beträgt.
4. Tabletten auf Basis einer Zusammensetzung gemäß Anspruch 1.
5. Verfahren zur Herstellung von Tabletten gemäß Anspruch 4, dadurch gekennzeichnet, daß man eine Zusammensetzung gemäß einem der Ansprüche 1, 2 oder 3 direkt oder nach vorheriger Trockengranulation zu Tabletten verpreßt.

**Ansprüche** (für den Vertragsstaat AT)

1. Verfahren zur Herstellung einer Zusammensetzung, enthaltend ein Gemisch aus Acesulfam oder einem physiologisch verträglichen Acesulfam-Salz, einer wasserlöslichen $CO_2$-liefernden Verbindung und gegebenenfalls einer physiologisch verträglichen, festen Säure, dadurch gekennzeichnet, daß man Gelatinepulver als Trockenbindemittel zusetzt.
2. Verfahren gemäß Anspruch 1, dadurch gekennzeichnet, daß man als $CO_2$-liefernde Verbindung Natriumhydrogencarbonat einsetzt.
3. Verfahren gemäß Anspruch 1 oder 2, dadurch gekennzeichnet, daß man 0,4 bis 5 % Gelatinepulver zusetzt.
4. Verfahren zur Herstellung von Tabletten, dadurch gekennzeichnet, daß man in einem weiteren Verfahrensschritt die Zusammensetzung, erhalten gemäß einem der Ansprüche 1 bis 3, direkt oder nach vorheriger Trockengranulation zu Tabletten verpreßt.

**Claims** (for the Contracting States : BE, CH, DE, FR, GB, IT, LI, NL, SE)

1. A composition containing acesulfam or a physiologically tolerable salt thereof, a water-soluble, $CO_2$-yielding compound and optionally a physiologically tolerable solid acid, characterized in that it contains gelatin powder as dry binder.
2. The composition as claimed in Claim 1, which comprises sodium bicarbonate as $CO_2$-yielding compound.
3. The composition as claimed in Claims 1 or 2, wherein the content of gelatin powder is from 0.4 to 5 %.
4. Tablets on the basis of a composition as claimed in Claim 1.
5. A process for the manufacture of tablets as claimed in Claim 4, which comprises compressing to tablets a composition as claimed in one of Claims 1, 2 or 3 either directly or after previous dry granulation.

**Claims** (for the Contracting State AT)

1. A process for the manufacture of a composition containing a mixture of cesulfam or a

physiologically tolerable salt thereof, a water-soluble, $CO_2$-yielding compound and optionally a physiologically tolerable solid acid, characterized in that gelatin powder is added as dry binder.

2. A process as claimed in Claim 1, wherein sodium bicarbonate is used as $CO_2$-yielding compound.

3. A process as claimed in Claims 1 or 2, wherein 0.4 to 5 % of gelatin powder is added.

4. A process for the manufacture of tablets which comprises in a further step compressing to tablets a composition obtained according to a process as claimed in one of Claims 1 to 3 either directly or after previous dry granulation.

**Revendications** (pour les Etats contractants : BE, CH, DE, FR, GB, IT, LI, NL, SE)

1. Composition contenant de l'acésulfame ou un sel d'acésulfame physiologiquement acceptable, un composé soluble dans l'eau dégageant $CO_2$ et éventuellement un acide solide physiologiquement acceptable, caractérisé en ce qu'il contient de la poudre de gélatine comme liant sec.

2. Composition selon la revendication 1, caractérisé en ce qu'il contient du bicarbonate de sodium comme composé dégageant du $CO_2$.

3. Composition selon l'une des revendications 1 et 2, caractérisé en ce que la teneur en poudre de gélatine atteint de 0,4 à 5 %.

4. Comprimés contenant une composition selon la revendication 1.

5. Procédé pour la production de composés selon la revendication 4, caractérisé en ce qu'on comprime en comprimés une composition selon l'une quelconque des revendications 1 à 3 directement ou après une granulation préalable à sec.

**Revendications** (pour l'Etat contractant AT)

1. Procédé pour la production d'une composition contenant un mélange d'acésulfame ou d'un sel d'acésulfame physiologiquement acceptable, d'un composé soluble dans l'eau dégageant du $CO_2$ et éventuellement d'un acide solide physiologiquement acceptable, caractérisé en ce qu'on ajoute de la poudre de gélatine comme liant sec.

2. Procédé selon la revendication 1, caractérisé en ce qu'on utilise du bicarbonate de sodium comme composé dégageant du $CO_2$.

3. Procédé selon l'une des revendications 1 et 2, caractérisé en ce qu'on ajoute de 0,4 à 5 % de poudre de gélatine.

4. Procédé pour la production de comprimés, caractérisé en ce que dans un autre stade opératoire, on comprime en comprimés la composition obtenue selon une des revendications 1 à 3, directement ou après une granulation préalable à sec.